# EUROPÄISCHE PATENTSCHRIFT

(11) **EP 4 345 352 B1**
(45) Veröffentlichungstag und Bekanntmachung des Hinweises auf die Patenterteilung: **13.08.2025**
(21) Anmeldenummer: 23198704.1
(22) Anmeldetag: 21.09.2023
(51) Int. Cl.: F16L 21/03, F16L 21/035, A61M 39/10, A61M 39/14, A61M 39/16

(54) **MEDIZINISCHE KONNEKTORVERBINDUNG MIT TOTRAUMARMER RADIALER DICHTUNG**
MEDICAL CONNECTOR CONNECTION WITH RADIAL SEAL WITH REDUCED DEAD SPACE
CONNECTEUR MÉDICAL AVEC JOINT RADIAL À FAIBLE ESPACE MORT

(30) Priorität: 30.09.2022 DE 102022125309
(43) Veröffentlichungstag der Anmeldung: 03.04.2024
(73) Patentinhaber: B. Braun Avitum AG, 34212 Melsungen (DE)
(72) Erfinder: KNIERIM, Michael, 34212 Melsungen-Schwarzenberg (DE)
(74) Vertreter: Winter, Brandl - Partnerschaft mbB

(56) Entgegenhaltungen:
- EP-A1- 0 406 144
- DE-A1- 2 855 646
- GB-A- 2 000 240

## Beschreibung

### Beschreibung

Die vorliegende Offenbarung betrifft eine totraumrarme radiale Dichtung, insbesondere für eine medizinische Konnektorverbindung mit einem männlichen Verbindungselement, einem weiblichen Verbindungselement und einem radialen Dichtungselement.

### Hintergrund der Offenbarung

Konnektorverbindungen mit einem ersten Verbindungselement, das in einem zweiten Verbindungselement angeordnet ist, sind hinlänglich bekannt. Kolben-Zylinder-Systeme mit einem (axial beweglichen) Kolben bzw. Stange und einem Zylindergehäuse sind hinlänglich bekannt und weisen üblicherweise ein radiales Dichtungselement auf, vorzugsweise in Form eines Dichtringes, welches an dem ersten Verbindungselement als Kolbenabdichtung oder an dem zweiten Verbindungselement als Stangenabdichtung ausgebildet ist. Dieses Dichtungselement hat die Aufgabe ein Eindringen bzw. ein Austreten von Fluid durch den radialen Spalt / Ritze, zwischen dem ersten Verbindungselement und dem zweiten Verbindungselement zu verhindern. Herkömmlicherweise, ist dieses Dichtungselement innerhalb einer Radialnut angeordnet, welche derart ausgebildet ist, dass bei dessen Montage bzw. während der bestehenden Konnektorverbindung eine ausreichende Flächenpressung erreicht wird, wodurch der gewünschte Abdichtungseffekt erzielt wird. Demnach weisen derartige Umlaufnuten meist etwas größere Dimensionen als das Dichtungselement selbst auf. Aufgrund der sich dadurch im montierten Zustand des Dichtungselements ausbildenden Toträume wird der Bereich dieser Abdichtungen vom Fluid meist schlecht durch- bzw. umflossen, was zu einer vermehrten Entstehung von Keimen bzw. einer vermehrten Ansammlung von Ablagerungen in diesen Bereichen führt.

Das Dokument CN 202 834 069 U beschreibt eine Vorrichtung mit einem gesonderten Desinfektionskanal innerhalb eines Zylindergehäuses, in welchem ein Kolben aufgenommen ist. Dieser stellt sicher, dass innere Komponente der Vorrichtung ausreichend umspült werden bzw. Ablagerungen ausreichend einfach abgeführt werden können. Dieser Desinfektionskanal steht dabei mit einer äußeren Fluidquelle in Verbindung.

Des Weiteren offenbart die EP 1 574 774 B1 eine Dichtungsanordnung mit einem Dichtring, welche innerhalb einer Nut angeordnet ist. Innerhalb der Nut bzw. an einer Flankenseite der Nut ist eine Einbuchtung angeordnet, die über einen Fluidkanal an eine Öffnung mit der Umgebung verbunden ist. Somit kann ungewollt innerhalb der Nut angesammeltes Fluid von dem Dichtring abgeführt und über die Öffnung an die Umgebung abgeführt werden.

Demnach sind aus dem Stand der Technik verschiedene Bauarten von Verbindungselementen mit einer totraumfreien Abdichtung bekannt, welche jedoch entweder zusätzliche Fluidquellen zum Reinigen der (inneren) Komponenten erfordern oder keine ausreichende Zuführung von Fluid an Dichtungselemente, insbesondere in Konnektorverbindungen, gewährleisten.

Aus der DE 28 55 646 A1, der EP 0 406 144 A1 und der GB 2 000 240 A sind jeweils Konnektorverbindungen mit einem ersten und einem zweiten Verbindungselement und einem Dichtungselement bekannt. Das Dichtungselement ist jeweils in einer Dichtungsnut aufgenommen. Die Dichtungsnut steht in Fluidkontakt zu einem Fluidkanal der Konnektorverbindung.

### Zusammenfassung der Offenbarung

Es sind die Aufgaben und Ziele der Offenbarung, die Nachteile aus dem Stand der Technik zu beheben oder wenigstens zu mildern, und insbesondere eine gegenüber dem Stand der Technik verbesserte medizinische Konnektorverbindung mit einem radialen Dichtungselement bereitzustellen, welches in bevorzugter Weise während des Betriebs von einem Fluid ausreichend umströmt oder umströmbar ist.

Die Aufgaben und Ziele werden hinsichtlich einer gattungsgemäßen medizinischen Konnektorverbindung offenbarungsgemäß durch den Gegenstand des Anspruchs 1 gelöst.

Die Offenbarung betrifft demzufolge eine medizinische Konnektorverbindung oder ein System aus zumindest zwei zueinander passenden Konnektoren zum Herstellen einer Fluidverbindung zwischen zwei Fluidleitungen. Die Konnektorverbindung weist ein erstes männliches Verbindungselement / Konnektorelement auf, das derart in einem zweiten weiblichen Verbindungselement / Konnektorelement angeordnet oder anordenbar ist, dass ein Fluidkanal des ersten Verbindungselements und ein Fluidkanal des zweiten Verbindungselements einen gemeinsamen Fluidkanal bilden. Die Konnektorverbindung weist ferner ein (Ab-)Dichtelement / Dichtungselement auf, welches innerhalb einer umlaufenden Nut an einer Außenseite des ersten Verbindungselements oder an einer Innenseite des zweiten Verbindungselements angeordnet ist und einen radialen Spalt zwischen dem zweiten Verbindungselement und dem ersten Verbindungselement abdichtet. Dabei weisen das erste Verbindungselement und/oder das zweite Verbindungselement zumindest eine Aussparung auf, welche den gemeinsamen Fluidkanal mit der umlaufenden Nut fluidisch verbindet bzw. fluidisch miteinander koppelt. Die zumindest eine Aussparung verläuft in einer axialen Richtung des ersten Verbindungselements.

In anderen Worten ausgedrückt weist ein medizinisches Kolben-Zylinder-System ein Zylindergehäuse, einen Kolben, welcher derart in dem Zylindergehäuse arretiert angeordnet oder anordenbar ist, dass ein Fluidkanal des Kolbens und ein Fluidkanal des Zylindergehäuses einen gemeinsamen Fluidkanal zum Herstellen einer Fluidverbindung bilden, und das (Ab-)Dichtelement / Dichtungselement auf. Das Kolben-Zylinder-System ist insbesondere die Konnektorverbindung, die einen männlichen Stecker und eine weibliche Dose aufweist. Männlich heißt in diesem Zusammenhang, dass der Stecker / das erste Verbindungselement konvex ausgebildet ist. Das weibliche Verbindungselement / die weibliche Dose ist im Wesentlichen konkav ausgebildet. Der Stecker wird dabei in die Dose eingeführt und in der Dose arretiert. Durch die fluiddichte Konnektorverbindung der beiden Verbindungselemente können beispielsweise zwei Fluidleitungen miteinander verbunden werden. Vorzugsweise kann das erste Verbindungselement in dem zweiten Verbindungselement derart arretiert / befestigt werden, dass eine fluiddichte Verbindung zwischen den Verbindungselementen entsteht.

Anders ausgedrückt, ist das Dichtungselement in der medizinischen Konnektorverbindung angeordnet, um einen radialen Spalt zwischen dem ersten und männlichen Verbindungselement und dem zweiten und weiblichen Verbindungselement abzudichten. Dieses Dichtungselement ist entweder in einer Radialnut entlang dem Umfang des ersten Verbindungselements oder in einer Radialnut auf Seiten des zweiten Verbindungselements, insbesondere an / auf dessen inneren Mantelfläche, vorgesehen. Außerdem ist zumindest eine Aussparung / Bohrung / Ausnehmung / Freimachung an dem ersten Verbindungselement und / oder an dem zweiten Verbindungselement derart angeordnet oder vorgesehen, dass das Fluid einen Bereich der umlaufenden Nut und des Dichtungselements ausreichend bzw. verbessert umfließt / durchfließt / durchströmt.

Nochmals anders ausgedrückt, ist die zumindest eine Aussparung an dem ersten Verbindungselement und / oder an dem zweiten Verbindungselement derart in axialer Richtung des ersten Verbindungselements / entlang der Axialachse des ersten Verbindungselements ausgerichtet / angeordnet, dass das Fluid bei bestehender (Konnektor-)Verbindung des ersten Verbindungselements mit dem zweiten Verbindungselement von dem Fluidkanal in die umlaufende Nut und somit zum Dichtungselement strömbar ist. Gleichzeitig kann das Fluid in axialer Richtung des ersten Verbindungselements wieder aus der umlaufenden Nut abfließen. Aufgrund einer solchen axialen Ausrichtung der zumindest einen Aussparung ist ein Zu- bzw. Abfließen des Fluids zum / vom Dichtungselement in axialer Richtung erzielbar.

Wenn Fluid durch den gemeinsamen Fluidkanal strömt, kann das Fluid durch die zumindest eine Aussparung zu der umlaufenden Nut und dem Dichtungselement strömen. Somit gelangt das Fluid während einer bestehenden Kolben-Zylinder-Verbindung bzw. während bestehenden (fluiddichten) Konnektorverbindung über die zumindest eine Aussparung an das Dichtungselement bzw. ist von diesem leichter abführbar, wodurch das Dichtungselement sowie die umlaufende Nut besser vom Fluid umspült werden. Nochmal anders ausgedrückt, ermöglicht die zumindest eine Aussparung eine bessere Durchströmung in dem Bereich des (Ab-)Dichtungselements, welches bspw. als O-Ring oder als Vierlippendichtring ausgebildet ist, insbesondere einer radialen Stangen- bzw. einer Kolbenabdichtung, durch das Fluid / Medium. Mithilfe der totraumarmen Konstruktion ist der Totraum im Bereich des Dichtungselements verkleinert / reduziert und das Fluid wird besser mitgerissen, wodurch das Risiko einer Entstehung / Ansammlung von Keimen reduziert ist. Zudem ist ein Vorgang einer Desinfektion, insbesondere im Bereich des Dichtungselements bzw. der umlaufenden Nut, leichter ausführbar.

Vorteilhafte Ausführungsformen sind Gegenstand der Unteransprüche und werden nachfolgend näher erläutert.

Vorzugsweise kann beim Zusammenstecken des ersten und des zweiten Verbindungselements bzw. beim Verbinden der beiden Verbindungselemente das erste Verbindungselement / der Kolben durch eine axiale Verschiebung in dem zweiten Verbindungselement / dem Zylindergehäuse ein Fluid verdrängen. Somit kann sich zwischen einer Stirnfläche des ersten Verbindungselements und einer Anschlagsfläche des zweiten Verbindungselements ein Verdrängungsraum bilden. Durch die zumindest eine Aussparung in dem ersten Verbindungselement und / oder in dem zweiten Verbindungselement kann der Verdrängungsraum mit der umlaufenden Nut fluidisch verbunden bzw. fluidisch miteinander gekoppelt sein.

Es ist ebenfalls denkbar, dass die Aussparung in einem Winkel zu der axialen Richtung des ersten Verbindungselements ausgebildet ist.

In einer ersten bevorzugten Ausführungsform der Offenbarung ist die zumindest eine Aussparung an einem bei bestehender Konnektorverbindung / im verbundenen Zustand dem zweiten Verbindungselement zugewandten ersten Endabschnitt / Endstück des ersten Verbindungselements zwischen der umlaufenden Nut und der Stirnfläche des ersten Verbindungselements angeordnet.

In anderen Worten, ist das Dichtungselement in einer ersten bevorzugten Ausführungsform in einer radial umlaufenden Nut an dem ersten Verbindungselement angeordnet und die zumindest eine Aussparung ist derart zwischen dieser umlaufenden Nut und einer Stirnfläche / Endfläche des dem zweiten Verbindungselement zugewandten ersten Endabschnitts ausgebildet. Die zumindest erste Aussparung ermöglicht eine Umströmung der umlaufenden Nut bzw. des darin aufgenommenen Dichtungselements. Diese bevorzugte erste Ausführungsform ist einfach und damit kostengünstig herstellbar, da die zumindest eine Aussparung durch Spritzguss im selben Herstellungsschritt wie das gesamte erste Verbindungselement gefertigt werden kann. Auf eine (Nach-)Bearbeitung des ersten Verbindungselements kann dabei verzichtet werden. Das erste Verbindungselement mitsamt der zumindest einen Aussparung kann somit in einem einzigen Herstellungsschritt gefertigt werden.

In einer weiteren bevorzugten Ausführungsform der Offenbarung ist zumindest eine der Aussparungen an dem ersten Endabschnitt des ersten Verbindungselements, der dem zweiten Verbindungselement zugewandt ist, zwischen der umlaufenden Nut und der Stirnfläche des ersten Verbindungselements und zumindest eine der Aussparungen an der Anschlagsfläche des zweiten Verbindungselements angeordnet.

Anders ausgedrückt, sind in dieser zweiten bevorzugten Ausführungsform Aussparungen an dem ersten Verbindungselement sowie (Gehäuse-)Aussparungen an dem zweiten Verbindungselement angeordnet, welche miteinander in Verbindung stehen bzw. ineinander überlaufen, um so einen Fluidfluss zwischen diesen Aussparungen herzustellen. Der ergänzende Effekt dieser Aussparungen an beiden Komponenten der Konnektorverbindung zueinander stellt eine ausreichende Umspülung des Dichtungselements bzw. Durchströmung der umlaufenden Nut sicher.

In einer weiteren bevorzugten Ausführungsform der Offenbarung ist zumindest eine der Aussparungen an einem inneren Mantelflächenabschnitt des zweiten Verbindungselements, welcher den ersten dem zweiten Verbindungselement zugewandten Endabschnitt umschließt, und zumindest eine der Aussparungen an der Anschlagsfläche des zweiten Verbindungselements angeordnet.

In anderen Worten, sind in dieser dritten Ausführungsform Aussparungen an dem zweiten Verbindungselement angeordnet, nämlich eine oder mehrere (Gehäuse-)Aussparungen an / auf der Anschlagsfläche des zweiten Verbindungselements sowie eine oder mehrere (Mantelflächen-)Aussparungen an einem inneren Mantelflächenabschnitt des zweiten Verbindungselements, welcher den dem zweiten Verbindungselement zugewandten Endabschnitt des ersten Verbindungselements umgreift / aufnimmt. Die Aussparungen an dem inneren Mantelflächenabschnitt erstrecken sich ebenfalls in axialer Richtung des ersten Verbindungselements und stehen zugleich mit den Aussparungen auf der Anschlagsfläche des zweiten Verbindungselements in Verbindung, sodass Fluid über diese Aussparungen zum Dichtungselement bzw. zur umlaufenden Nut strömen kann. In dieser Ausführung sind keine zusätzlichen Aussparungen an / auf dem ersten Verbindungselement notwendig. Demnach kann die Gestaltung des ersten Verbindungselements einfach gehalten werden.

In einer weiteren bevorzugten Ausführungsform der Offenbarung ist zumindest eine der Aussparungen an einem inneren Mantelflächenabschnitt des zweiten Verbindungselements, welches einen ersten dem zweiten Verbindungselement zugewandten Endabschnitt des ersten Verbindungselements umgibt, und zumindest eine der Aussparungen an dem Endabschnitt des ersten Verbindungselements angeordnet.

Anders ausgedrückt, weist das zweite Verbindungselement in dieser vierten bevorzugten Ausführungsform eine oder mehrere, insbesondere sich in Richtung einer Längsachse des ersten Verbindungselements erstreckende, (Mantelflächen-)Aussparungen an dem inneren Mantelflächenabschnitt auf, welcher den dem zweiten Verbindungselement zugewandten Endabschnitt des ersten Verbindungselements umgreift / aufnimmt. Gleichzeitig ist eine oder mehrere Aussparungen an den dem zweiten Verbindungselement zugewandten Endabschnitt ausgebildet, welche in die entsprechende Aussparung oder Aussparungen auf der Mantelinnenfläche des zweiten Verbindungselements überlaufen bzw. auslaufen. Auch diese vierte Ausführungsform der Konnektorverbindung ermöglicht ein Um- / Durchströmen des Dichtungselements bzw. der, insbesondere an dem zweiten Verbindungselement ausgebildeten, umlaufenden Nut.

In einem weiteren bevorzugten Aspekt der Offenbarung, weist der erste Endabschnitt einen geringeren Außendurchmesser auf als der restliche Teil des ersten Verbindungselements. Anders ausgedrückt, ist der Außendurchmesser des ersten Verbindungselements in einem Endstück / Abschnitt reduziert, welches dem zweiten Verbindungselement zugewandt ist. Dies bietet eine zusätzliche Möglichkeit einen ungehinderten Fluidstrom zwischen dem Dichtungselement und dem Fluidkanal der Konnektorverbindung zu erzielen. Das Fluid ist dann in der Lage am Außenumfang des entsprechenden Endabschnitts des ersten Verbindungselements vorbeizufließen und somit auch einfacher in die angrenzenden Aussparungen an dem ersten Verbindungselement und / oder an dem zweiten Verbindungselement zu fließen. Die Umströmung / Anströmung des Dichtungselements ist zusätzlich verbessert / vereinfacht.

In einem weiteren bevorzugten Aspekt der Offenbarung sind die Aussparungen über den Umfang des ersten Verbindungselements und / oder über den Innenumfang des zweiten Verbindungselements, insbesondere in gleichen Abständen voneinander, verteilt angeordnet.

Die Aussparungen können aber auch in unregelmäßigen Abständen über den Umfang des ersten Verbindungselements und / oder über den Innenumfang des zweiten Verbindungselements verteilt sein.

In einem weiteren bevorzugten Aspekt der Offenbarung weist der Fluidkanal an der Anschlagsfläche des zweiten Verbindungselements eine trichterförmige Öffnung auf. Das heißt, dass sich die Öffnung des Fluidkanals im Inneren des zweiten Verbindungselements trichterförmig in Richtung der Anschlagsfläche bzw. in Richtung der Stirnfläche des ersten Verbindungselements aufweitet / verbreitet. Hierdurch ist eine verbesserte Verteilung / Ausbreitung des Fluids in die entsprechenden Aussparungen an dem ersten Verbindungselement und / oder an dem zweiten Verbindungselement möglich. Demnach ist es möglich, die Aussparungen kleiner bzw. kürzer zu dimensionieren.

In einem weiteren bevorzugten Aspekt der Offenbarung weist das erste Verbindungselement an dessen Stirnfläche eine umlaufende Fase auf. In anderen Worten, ist am Übergang der Stirnfläche des ersten Verbindungselements, welche dem Fluidkanal bzw. der Anschlagsfläche des zweiten Verbindungselements zugewandt ist, zur Mantelfläche des ersten Verbindungselements eine umlaufende Fase ausgebildet. Durch diese umlaufende Fase ist das erste Endstück besser vom Fluid umströmbar, wodurch eine Anströmung des radialen Dichtungselements erleichtert ist. Weiter anders ausgedrückt, weisen die Flussdurchmesser der sich fügenden Komponenten umlaufende Fasen auf.

### Kurzbeschreibung der Figuren

Die Offenbarung sowie das technische Umfeld werden nachfolgend anhand der Figuren näher erläutert. Es ist darauf hinzuweisen, dass die Offenbarung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und Erkenntnissen aus der vorliegenden Beschreibung und/oder Figuren zu kombinieren. Insbesondere ist darauf hinzuweisen, dass die Figuren und insbesondere die dargestellten Größenverhältnisse nur schematisch sind. Gleiche Merkmale werden mit denselben Bezugszeichen referenziert. Außerdem wird darauf hingewiesen, dass die Merkmale der einzelnen Ausführungsformen untereinander ausgetauscht werden sowie in einer bestimmten Kombination auftreten können.

Die Offenbarung wird nachfolgend anhand bevorzugter Ausführungsbeispiele mit Hilfe von Figuren näher erläutert. Es zeigen:
Figuren 1A - 1C eine schematische isometrische Seitenansicht auf einen einem zweiten Verbindungselement zugewandten ersten Endabschnitt eines ersten Verbindungselements sowie schematische Längsschnittansichten des zweiten Verbindungselements und der Konnektorverbindung gemäß einer ersten vorteilhaften Ausführungsform;
Figuren 2A und 2B eine schematische isometrische Seitenansicht auf den ersten Endabschnitt sowie eine schematische Längsschnittansicht der Konnektorverbindung gemäß einer zweiten vorteilhaften Ausführungsform;
Figuren 3A - 3C eine schematische isometrische Seitenansicht auf den ersten Endabschnitt sowie schematische Längsschnittansichten des zweiten Verbindungselements und der Konnektorverbindung gemäß einer dritten vorteilhaften Ausführungsform;
Figuren 4A - 4C schematische Längsschnittansichten des Endabschnitts, des zweiten Verbindungselements und der Konnektorverbindung gemäß einer vierten vorteilhaften Ausführungsform; und
Figuren 5A - 5C schematische Längsschnittansichten des Endabschnitts, des zweiten Verbindungselements und der Konnektorverbindung gemäß einer letzten, fünften Ausführungsform.

### Detaillierte Beschreibung der Figuren

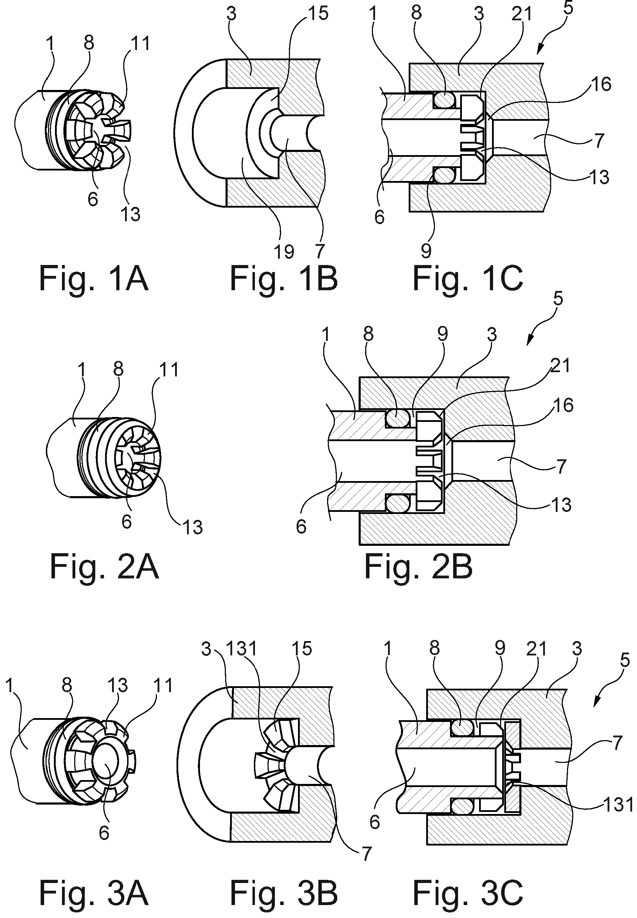

Figur 1A zeigt eine schematische isometrische Seitenansicht auf einen ersten Endabschnitt des ersten und männlichen Verbindungselements 1, Figur 1B zeigt eine schematische Längsschnittansicht eines zweiten und weiblichen Verbindungselements 3 und Figur 1C zeigt eine schematische Längsschnittansicht einer Konnektorverbindung 5 gemäß einer ersten Ausführungsform, die aus den jeweiligen in den Figuren 1A und 1B gezeigten Komponenten ausgebildet ist. Das erste Verbindungselement 1 weist einen entlang dessen Achse verlaufenden Fluidkanal 6 und an einem Endstück des ersten Verbindungselements 1 ein radiales Dichtungselement / Dichtring 8 auf, welches in einer umlaufenden Nut 9 an dem ersten Verbindungselement 1 angeordnet ist. Eine Stirnfläche 11 des ersten Verbindungselements 1, welche eine Endfläche senkrecht zur Achse des ersten Verbindungselements 1 bildet und in welche der Fluidkanal 6 ausläuft, weist zudem eine Vielzahl an Aussparungen 13 auf, die entlang dem Umfang des ersten Verbindungselements 1 verteilt angeordnet sind. Ferner, sind die Aussparungen 13 gleichmäßig voneinander beabstandet, weisen also gleich große Abstände entlang des Umfangs des ersten Verbindungselements auf. Dabei sind aber durchaus auch Ausführungsformen der Erfindung möglich, bei denen die Aussparungen ungleichmäßig beabstandet, also unterschiedliche Abstände entlang des Umfangs des ersten Verbindungselements 1 aufweisen. Die Aussparungen 13 sind in der dargestellten Ausführungsform derart ausgebildet, dass diese in die umlaufende Nut 9 münden / auslaufen, in welcher das Dichtungselement 8 angeordnet ist, und gleichzeitig die Wand des Fluidkanals 6 im Bereich der Stirnseite 11 durchbrechen. Das in den Figuren 1B und 1C abgebildete zweite Verbindungselement 3 weist einen hohlzylinderförmigen Abschnitt für die Aufnahme des ersten Verbindungselements 1 auf, der von einer Anschlagsfläche 15 begrenzt ist. Ferner, ist eine trichterförmige Öffnung 16 an der Anschlagsfläche 15 ausgebildet, welche in einen Fluidkanal 7 des zweiten Verbindungselements 3 mündet. Zwischen der Stirnfläche 11 und der Anschlagsfläche 15 ist eine (Fluid-)Kammer 19 ausgebildet. Insbesondere in der Fig. 1C ist die Vielzahl der Aussparungen 13 an dem ersten Verbindungselement 1 erkennbar, durch welche die umlaufende Nut 9 bzw. eine Seite des Dichtungselements 8 mit der Fluidkammer 19 und somit dem Fluidkanal 7 fluidisch in Verbindung steht. Des Weiteren, weist das erste Verbindungselement 1 eine umlaufende Fase auf, welche einen Übergang zwischen der Stirnseite 11 und der Umfangsseite des ersten Verbindungselements 1 bildet.

Figur 2A zeigt das erste Verbindungselement 1 in einer weiteren vorteilhaften Ausführungsform und Figur 2B zeigt eine zweite Ausführungsform der Konnektorverbindung 5 mit dem ersten Verbindungselement 1 aus der Figur 2A sowie dem zweiten Verbindungselement 3 aus der Figur 1B. In dieser zweiten Ausführungsform sind die Aussparungen 13 an dem ersten Verbindungselement 1 derart angeordnet, dass sie die Außenwand des ersten Verbindungselements 1 nicht durchbrechen, sondern innenseitig an dem ersten Verbindungselement 1 anliegen und lediglich die innere Wand des Fluidkanals 6 im Bereich der Stirnseite 11 durchbrechen. Die Aussparungen 13 erstrecken sich axial durch das erste Verbindungselement 1 bis zur umlaufenden Nut 9, in welcher das Dichtungselement 8 angeordnet ist. Somit ist die umlaufende Nut 9 inklusive dem Dichtungselement 8 mit dem Fluidkanal 7 des zweiten Verbindungselements 3 fluidisch miteinander verbunden.

Figur 3A zeigt eine schematische isometrische Seitenansicht auf den ersten Endabschnitt des ersten Verbindungselements 1 in einer weiteren Ausführungsform und Figuren 3B und 3C zeigen eine schematische Längsschnittansicht des zweiten Verbindungselements 3 und der Konnektorverbindung 5 gemäß einer dritten vorteilhaften Ausführungsform. Das erste Verbindungselement 1 dieser Ausführungsform unterscheidet sich von den oben beschriebenen Ausführungsformen in der Hinsicht, dass die Vielzahl der Aussparungen 13 die Wand des Fluidkanals 6 nicht durchbrechen bzw. nicht in den Fluidkanal 6 des ersten Verbindungselements 1 hineinragen. Die Aussparungen 13 sind in Form von außenliegenden Durchbrüchen angeordnet. Die ermöglicht einen Fluidfluss von der Stirnseite 11 in Richtung des Dichtungselement 8 bzw. der umlaufenden Nut 9. Das zweite Verbindungselement 3 dieser dritten Ausführungsform weist an dessen Anschlagsfläche 15 ebenfalls eine Vielzahl weiterer Aussparungen 131 auf, die von dem Fluidkanal 7 ausgehen und sich in Richtung der Innenwand des zweiten Verbindungselements 3 erstrecken. Die Konnektorverbindung 5 gemäß der dritten Ausführungsform in Figur 3C zeigt, dass die Aussparungen 11 an dem ersten Verbindungselement 1 und die Aussparungen 131 an der Innenseite des zweiten Verbindungselements 3 derart ausgebildet sind, dass das Dichtungselement 8 bzw. die umlaufende Nut 9 an dem ersten Verbindungselement 1 sowie der Fluidkanal 7 fluidisch miteinander in Verbindung stehen. Fluid fließt also über die Aussparungen 11 und 131 zu dem Dichtungselement 8 bzw. von diesem ab.

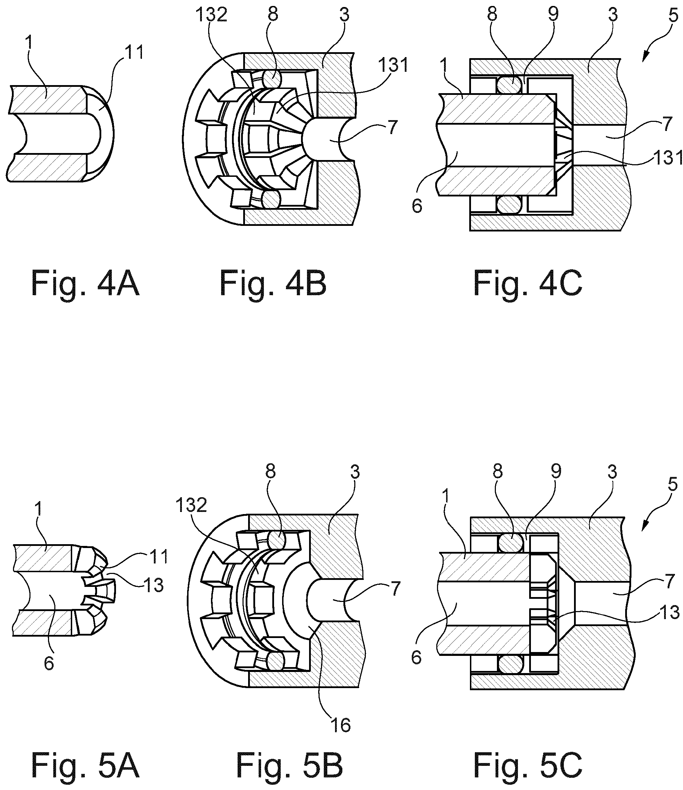

Die Figuren 4A und 4B zeigen eine schematische Längsschnittansicht des ersten Endabschnitts und des zweiten Verbindungselements 3 einer weiteren Ausführungsform. Figur 4C zeigt eine schematische Längsschnittansicht der Konnektorverbindung 5 gemäß einer vierten vorteilhaften Ausführungsform, das aus den der in den Figuren 4A und 4B dargestellten Komponenten zusammengesetzt ist. Das radiale Dichtungselement 8 ist dabei an der Innenseite des zweiten Verbindungselements 3, in Form einer Stangenabdichtung angeordnet. In dieser Ausführungsform weist lediglich das zweite Verbindungselement 3 Aussparungen 131 und 132 auf. Dabei sind die Aussparungen 131 wie in der Ausführungsform der Figuren 3B und 3C an der Anschlagsfläche 15 und die Aussparungen 132 an der inneren Mantelfläche des zweiten Verbindungselements 3 angeordnet, welche das erste Verbindungselement 1 im Betriebszustand umgibt. Die Aussparungen 131, 132 sind dabei derart ausgebildet, dass sie aneinander angrenzen und jeweils ineinander überlaufen / verlaufen. Hierdurch entsteht die fluidische Verbindung zwischen dem Dichtungselement 8 und dem Fluidkanal 7.

Die Figuren 5A und 5B zeigen schematische Längsschnittansichten des ersten Endabschnitts des ersten Verbindungselements 1 und des zweiten Verbindungselements 3 einer weiteren Ausführungsform. Figur 5C zeigt eine schematische Längsschnittansicht der Konnektorverbindung 5 gemäß einer letzten, fünften Ausführungsform, das aus den der in den Figuren 5A und 5B dargestellten Komponenten zusammengesetzt ist. Das Dichtungselement 8 ist wie in der Ausführungsform nach Fig. 4B an der Innenseite des zweiten Verbindungselements 3, in Form einer Stangenabdichtung angeordnet und das erste Verbindungselement 1 weist Aussparungen 13 gemäß der in Fig. 1A aufgezeigten Ausführungsform auf. Auf Seiten des zweiten Verbindungselements 3 sind lediglich Aussparungen 132 an der inneren Mantelfläche des Zylindergehäuses 3 angeordnet. Durch die Ausbildung der Aussparungen 13 an dem ersten Verbindungselement 1 ist eine fluidische Verbindung zwischen dem Fluidkanal 7 und dem Dichtungselement 8 ermöglicht, da die Aussparungen 13 des ersten Verbindungselements 1 an den Aussparungen 132 des zweiten Verbindungselements 3 angrenzen. Zudem weist der Fluidkanal 7 des Zylindergehäuses 3 eine trichterförmige Öffnung 16 auf.

### Bezugszeichenliste

- 1: erstes Verbindungselement
- 3: zweites Verbindungselement
- 5: Konnektorverbindung
- 6: Fluidkanal des ersten Verbindungselements
- 7: Fluidkanal des zweiten Verbindungselements
- 8: Dichtungselement
- 9: Nut
- 11: (Kolben-)Stirnfläche
- 13; 131; 132: Aussparung
- 15: Anschlagsfläche
- 16: trichterförmige Öffnung
- 19: Fluidkammer
- 21: Fase

## Patentansprüche

1. Medizinische Konnektorverbindung (5) zum Herstellen einer Fluidverbindung zwischen zwei Fluidleitungen mit einem männlichen ersten Verbindungselement (1) und einem weiblichen zweiten Verbindungselement (3),
wobei das erste Verbindungselement (1) derart in dem zweiten Verbindungselement (3) angeordnet oder anordenbar ist, dass ein Fluidkanal (6) des ersten Verbindungselements (1) und ein Fluidkanal (7) des zweiten Verbindungselements (3) einen gemeinsamen Fluidkanal bilden,
und einem Dichtungselement (8), welches innerhalb einer umlaufenden Nut (9) an einer Außenseite des ersten Verbindungselements (1) oder an einer Innenseite des zweiten Verbindungselements (3) angeordnet ist und einen radialen Spalt zwischen dem zweiten Verbindungselement (3) und dem ersten Verbindungselement (1) abdichtet, wobei
das erste Verbindungselement (1) und/oder das zweite Verbindungselement (3) zumindest eine Aussparung (13; 131; 132) aufweisen, welche den gemeinsamen Fluidkanal mit der umlaufenden Nut (9) fluidisch verbindet,
**dadurch gekennzeichnet, dass**
die zumindest eine Aussparung (13; 131; 132) in einer axialen Richtung des ersten Verbindungselements (1) ausgerichtet ist.

2. Konnektorverbindung (5) nach Anspruch 1, **dadurch gekennzeichnet, dass** die zumindest eine Aussparung (13) an einem dem zweiten Verbindungselement (3) zugewandten ersten Endabschnitt des ersten Verbindungselements (1) zwischen der umlaufenden Nut (9) und einer Stirnfläche (11) des ersten Verbindungselements (1) angeordnet ist.

3. Konnektorverbindung (5) nach Anspruch 1, **dadurch gekennzeichnet, dass** zumindest eine der Aussparungen (13) an einem dem zweiten Verbindungselement (3) zugewandten ersten Endabschnitt des ersten Verbindungselements (1) zwischen der umlaufenden Nut (9) und einer Stirnfläche (11) des ersten Verbindungselements (1) und zumindest eine der Aussparungen (131) an einer Anschlagsfläche (15) des zweiten Verbindungselements (3) angeordnet sind.

4. Konnektorverbindung (5) nach Anspruch 1, **dadurch gekennzeichnet, dass** zumindest eine der Aussparungen (132) an einem inneren Mantelflächenabschnitt des zweiten Verbindungselements (3), welcher einen ersten dem zweiten Verbindungselement (3) zugewandten Endabschnitt des ersten Verbindungselements (1) umschließt, und zumindest eine der Aussparungen (131) an einer Anschlagsfläche (15) des zweiten Verbindungselements (3) angeordnet sind.

5. Konnektorverbindung (5) nach Anspruch 1, **dadurch gekennzeichnet, dass** zumindest eine der Aussparungen (132) an einem inneren Mantelflächenabschnitt des zweiten Verbindungselements (3), welcher einen ersten dem zweiten Verbindungselement (3) zugewandten Endabschnitt des ersten Verbindungselements (1) umschließt, und zumindest eine der Aussparungen (13) an dem Endabschnitt des ersten Verbindungselements (1) angeordnet sind.

6. Konnektorverbindung (5) nach einem der Ansprüche 2 bis 5, **dadurch gekennzeichnet, dass** der dem zweiten Verbindungselement (3) zugewandte erste Endabschnitt des ersten Verbindungselements (1) einen geringeren Außendurchmesser als der restliche Teil des ersten Verbindungselements (1) aufweist.

7. Konnektorverbindung (5) nach einem der vorhergehenden Ansprüche, **dadurch gekennzeichnet, dass** die Aussparungen (13; 131; 132) über den Umfang des ersten Verbindungselements (1) und/oder über den Innenumfang des zweiten Verbindungselements (3), insbesondere in gleichen Abständen voneinander verteilt, angeordnet sind.

8. Konnektorverbindung (5) nach einem der Ansprüche 3 bis 7, **dadurch gekennzeichnet, dass** der Fluidkanal (7) an der Anschlagsfläche (15) eine trichterförmige Öffnung (16) aufweist.

9. Konnektorverbindung (5) nach einem der vorhergehenden Ansprüche, **dadurch gekennzeichnet, dass** das erste Verbindungselement (1) an dessen Stirnfläche (11) eine umlaufende Fase (21) aufweist.

## Claims

1. A medical connector link (5) for establishing a fluid connection between two fluid lines with a male first link element (1) and a female second link element (3),
wherein the first link element (1) is arranged or arrangeable in the second link element (3) such that a fluid passage (6) of the first link element (1) and a fluid passage (7) of the second link element (3) form a common fluid passage,
and a seal element (8), which is arranged within a circumferential groove (9) on an outer side of the first link element (1) or on an inner side of the second link element (3) and seals a radial gap between the second link element (3) and the first link element (1), wherein
the first link element (1) and/or the second link element (3) have at least one recess (13; 131; 132) that fluidically connects the common fluid passage with the circumferential groove (9),
**characterized in that**
the at least one recess (13; 131; 132) extends in an axial direction of the first link element (1).

2. The connector link (5) according to claim 1, **characterized in that** the at least one recess (13) is arranged at a first end portion of the first link element (1) facing the second link element (3) between the circumferential groove (9) and a front face (11) of the first link element (1).

3. The connector link (5) according to claim 1, **characterized in that** at least one of the recesses (13) is arranged on a first end portion of the first link element (1) facing the second link element (3), between the circumferential groove (9) and a front face (11) of the first link element (1), and at least one of the recesses (131) is arranged at a stop surface (15) of the second link element (3).

4. The connector link (5) according to claim 1, **characterized in that** at least one of the recesses (132) is arranged on an inner lateral surface portion of the second link element (3) that surrounds a first end portion of the first link element (1) facing the second link element (3), and at least one of the recesses (131) is arranged on a stop surface (15) of the second link element (3).

5. The connector link (5) according to claim 1, **characterized in that** at least one of the recesses (132) is arranged at an inner lateral surface portion of the second link element (3), which encloses a first end portion of the first link element (1) facing the second link element (3), and at least one of the recesses (13) is arranged at the end portion of the first link element (1).

6. The connector link (5) according to one of claims 2 to 5, **characterized in that** the first end portion of the first link element (1) facing the second link element (3) has a smaller outer diameter than the rest of the first link element (1).

7. The connector link (5) according to one of the preceding claims, **characterized in that** the recesses (13; 131; 132) are distributed over the circumference of the first link element (1) and/or over the inner circumference of the second link element (3), in particular at equal distances from each other.

8. The connector link (5) according to one of claims 3 to 7, **characterized in that** the fluid passage (7) has a funnel-shaped opening (16) at the stop surface (15).

9. The connector link (5) according to one of the preceding claims, **characterized in that** the first link element (1) has a circumferential chamfer (21) on its front face (11).

## Revendications

1. Connexion de connecteur médical (5) pour établir une connexion fluidique entre deux conduites de fluide avec un premier élément de connexion mâle (1) et un second élément de connexion femelle (3),
dans laquelle le premier élément de connexion (1) est disposé ou peut être disposé dans le second élément de connexion (3) de sorte qu'un canal de fluide (6) du premier élément de connexion (1) et un canal de fluide (7) du second élément de connexion (3) forment un canal de fluide commun,
et un élément d'étanchéité (8) qui est disposé à l'intérieur d'une rainure périphérique (9) au niveau d'un côté extérieur du premier élément de connexion (1) ou au niveau d'un côté intérieur du second élément de connexion (3) et étanchéifie une fente radiale entre le second élément de connexion (3) et le premier élément de connexion (1),
le premier élément de connexion (1) et/ou le second élément de connexion (3) présentent au moins un évidement (13 ; 131 ; 132) qui connecte fluidiquement le canal de fluide commun à la rainure périphérique (9),
**caractérisée en ce que**
le au moins un évidement (13 ; 131 ; 132) est orienté dans une direction axiale du premier élément de connexion (1).

2. Connexion de connecteur (5) selon la revendication 1, **caractérisée en ce que** le au moins un évidement (13) est disposé au niveau d'une première section d'extrémité du premier élément de connexion (1) tournée vers le second élément de connexion (3) entre la rainure périphérique (9) et une surface avant (11) du premier élément de connexion (1).

3. Connexion de connecteur (5) selon la revendication 1, **caractérisée en ce qu'**au moins l'un des évidements (13) est disposé au niveau d'une première section d'extrémité du premier élément de connexion (1) tournée vers le second élément de connexion (3) entre la rainure périphérique (9) et une surface avant (11) du premier élément de connexion (1), et au moins un des évidements (131) est disposé au niveau d'une surface de butée (15) du second élément de connexion (3).

4. Connexion de connecteur (5) selon la revendication 1, **caractérisée en ce qu'**au moins l'un des évidements (132) est disposé au niveau d'une section de surface d'enveloppe intérieure du second élément de connexion (3), section qui renferme une première section d'extrémité du premier élément de connexion (1) tournée vers le second élément de connexion (3), et au moins l'un des évidements (131) est disposé au niveau d'une surface de butée (15) du second élément de connexion (3).

5. Connexion de connecteur (5) selon la revendication 1, **caractérisée en ce qu'**au moins l'un des évidements (132) est disposé au niveau d'une section de surface d'enveloppe intérieure du second élément de connexion (3), section qui renferme une première section d'extrémité du premier élément de connexion (1) tournée vers le second élément de connexion (3), et au moins l'un des évidements (13) est disposé au niveau de la section d'extrémité du premier élément de connexion (1).

6. Connexion de connecteur (5) selon l'une quelconque des revendications 2 à 5, **caractérisée en ce que** la première section d'extrémité du premier élément de connexion (1) tournée vers le second élément de connexion (3) présente un diamètre extérieur inférieur à la partie restante du premier élément de connexion (1).

7. Connexion de connecteur (5) selon l'une quelconque des revendications précédentes, **caractérisée en ce que** les évidements (13 ; 131 ; 132) sont disposés sur la périphérie du premier élément de connexion (1) et/ou sur la périphérie intérieure du second élément de connexion (3), en particulier répartis à intervalles réguliers les uns des autres.

8. Connexion de connecteur (5) selon l'une quelconque des revendications 3 à 7, **caractérisée en ce que** le canal de fluide (7) présente une ouverture en forme d'entonnoir (16) au niveau de la surface de butée (15).

9. Connexion de connecteur (5) selon l'une quelconque des revendications précédentes, **caractérisée en ce que** le premier élément de connexion (1) présente un chanfrein périphérique (21) au niveau de sa surface frontale (11).
